# EUROPÄISCHE PATENTANMELDUNG

(11) **EP 1 524 315 A1**
(43) Veröffentlichungstag der Anmeldung: **20.04.2005**
(21) Anmeldenummer: 03450232.8
(22) Anmeldetag: 17.10.2003
(51) Int. Cl.: C12M 1/113

(54) **Rohrfermenter**

(71) Anmelder: Ing. Friedrich Bauer GmbH, 3373 Kemmelbach (AT)
(72) Erfinder: Bauer, Friedrich, 3373 Kemmelbach (AT)

(57) **Zusammenfassung**

Rohrfermenter zur Fermentation organischen Materials unter Erzeugung von Biogas, mit einem Eintrag 5 für das organische Material und einem Austrag 6 für das organische Material und für Biogas, einem Rührwerk 8a,8b, einer mit diesem umlaufenden Heizung sowie einem Gritaustrag. Die Heizung umfaßt zwei oder mehrere voneinander unabhängig steuerbare Heizwerke 18a,18b. Jedes der Heizwerke 18a,18b ist einem vorwählbaren Bereich des korrespondierenden Rührwerkes 8a,8b zugeordnet.

Die Reaktorwelle 9 des Rohrfermenters 2 besteht aus wenigstens zwei oder mehreren Wellenabschnitten 13a, 13b, 13c, 13d.

## Beschreibung

Die Erfindung betrifft einen Rohrfermenter zur Fermentation organischen Materials unter Erzeugung von Biogas, mit einem Eintrag für das organische Material und einem Austrag für das organische Material und Biogas, einem Rührwerk, einer mit diesem umlaufenden Heizung sowie einem Gritaustrag.
Biogas entsteht in einem mehrstufigen Prozeß der Vergärung oder Faulung von organischem Material unter Einsatz von anaeroben Mikroorganismen. Der biologische Abbau von organischem Material zerlegt hochmolekulare organische Substanzen in niedermolekulare Verbindungen. Dabei entsteht neben Biogas ein Gemisch aus Wasser, nicht abgebauten organischen Substanzen [ i.a. zellulosereiche Substanzen ] und nicht organischen Substanzen.
Wesentliche Bestandteile des Biogases sind Methan und Kohlendioxid.
Die Phasen des mehrstufigen Prozesses zur Erzeugung von Biogas sind die Hydrolyse, die Säurebildung, die Essigsäurebildung sowie die Methanbildung.
Wesentlicher Bestandteil zur Erzeugung von Biogas ist der Reaktor oder Fermenter.
Reaktoren zur Erzeugung von Biogas durch Fermentation organischen Materials sind allgemein bekannt.
Aus der eigenen AT 405 185 B ist ein Rohrfermenter bekannt, bei dem die Heizung an das Rührwerk zur Erzeugung einer kontinuierlichen Propfenströmung gekoppelt ist und sich mit diesem dreht. Im Bereich des Domes, also im oberen Bereich des Rohrfermenters ist ein gemeinsamer Austrag von Biogas und fermentiertem Substrat vorgesehen. Das Rührwerk selbst unterliegt einer Bewegungskontrolle zur Regelung der Beschickung des Rohrfermenters. Der Eintrag des organischen Materials erfolgt in an sich bekannter Weise im unteren Anfangsbereich des Rohrfermenters.
Trotz aller Vorteile dieses Aufbaus, können bei der Abarbeitung von biogenem Material inkonsistente Verhältnisse entstehen, insbesondere im Bereich der Wärmeübergänge, vor allem bei längeren Rohrfermentem. Daraus resultiert eine ungleichmäßige Abarbeitung des biogenen Materials, welche sich auf die Ergebnisrate des erzeugten Biogases niederschlägt.
Darüberhinaus führt der bekannte Eintrag des organischen Materials bei einer Reihe von Materialien zu Einmischproblemen.
Oben genannte Nachteile führen oft auch zu einem Abriß der angestrebten kontinuierlichen Pfropfenströmung.

Aufgabe der Erfindung ist es, die Nachteile bekannter Rohrfermenter zu vermeiden und Voraussetzungen zur konstanten Abarbeitung biogenen Materials zu schaffen. Ein wesentliches Kennzeichen dafür ist die Möglichkeit eines sowohl mesophilen als auch thermophilen Betriebes.
Die Erfindung löst die Aufgabe dadurch, daß die Heizung zwei oder mehrere voneinander unabhängig steuerbare Heizwerke umfaßt und daß jedes der Heizwerke einem vorwählbaren Bereich des korrespondierenden Rührwerkes zugeordnet ist.
Daraus resultiert ein kontrollierbares Handling auftretender Wärmeschwankungen, insbesondere in der Prozeßwärme. Sowohl eine linear steigende als auch exponentiale Temperaturkurve kann durch den Einsatz von zwei oder mehreren unabhängig steuerbaren Heizwerken gesteuert bzw. ein- oder nachreguliert werden.
Die erfindungsgemäße Lösung ermöglicht weiters ein Ergänzen der Abstrahlungswärme. Auch ein Durchwärmen des Substrates - in Abhängigkeit vom Prozeß - von vorne nach hinten [ oder umgekehrt ] ist möglich.

Der erfindungsgemäß eingesetzte Rohrfermenter umfaßt ein Rührwerk mit einer Reaktorwelle, einer Vielzahl über die Längserstreckung der Welle gegeneinander versetzter Paddel sowie Rührschaufeln im Bereich der Reaktorwand.
Dabei umfaßt die Reaktorwelle wenigstens zwei- oder mehrere Wellenabschnitte. Diese können beliebig, auch unabhängig voneinander angesteuert bzw. mit Information für die an ihnen angeordneten Elemente versorgt werden.
Nach weiteren Merkmalen der Erfindung ist vorgesehen, daß die Enden jeweils zweier benachbarter Wellenabschnitte von einem gemeinsamen Stützlager geführt sind und daß die Enden der aus Wellenabschnitten gebildeten Reaktorwellen ebenfalls in Stützagern geführt sind.
Durch die Trennung der Antriebe kann ein genau definiertes Abarbeiten des biogenen Materials durchgeführt werden. Die Schaffung konstanter thermischer Verhältnisse ist somit problemlos möglich. Weiters löst dieses Merkmal der Erfindung die Ausbildung besserer Wärmeübergänge.
Durch die Mehrteilung der Reaktorwelle sowie die Entkopplung der Antriebe ist sowohl mesophiler als auch thermophiler Betrieb möglich.
Idealerweise befindet sich in den vorderen 20% der Rohrfermenters die Säuerungsphase, in den verbleibenden hinteren 80% erfolgt die Methanisierung.
Nach einer Weiterbildung der Erfindung sind zwei unabhängige Reaktorwellen vorgesehen, wobei jede Reaktorwelle einen unabhängigen Wellenantrieb aufweist.
Erfindungsgemäß erfolgt eine Zuordnung der Heizwerke zur Reaktorwelle dermaßen, daß eines der Heizwerke im Eintrittsbereich des Rohrfermenters vorgesehen ist und daß das zweite sowie jedes weitere Heizwerk im an den Eintrittsbereich anschließenden Bereich des Rohrfermenters vorwählbar vorgesehen ist.
Sämtliche Heizwerke sind unabhängig steuerbar und können somit zufolge ihrer wählbaren Anordnung bezogen auf den jeweils gefahrenen Prozeß die erforderliche Konstanz der gewünschten Heizungsart des abzuarbeitenden Biosubstrates [ entsprechend der vorgewählten Heizkurve ] garantieren.
Nach einem anderen Merkmal der Erfindung ist vorgesehen, daß der Gritaustrag außerhalb des Eintragsbereiches angeordnet ist und daß der Gritaustrag eine aus dem Rohrfermenter schräg nach unten verlaufende Austragstrecke umfaßt.
Durch dieses Merkmal wird der Gritaustrag aus dem Bereich der Materialeinbringung genommen. Dadurch wird auch die Materialentnahme zur Prüfung des Substrates vereinfacht. Die schräg nach unten verlaufende Austragsstrecke ermöglicht eine einfachere Entnahme und Reinigung.
Bei Fermentern mit großer Länge ist die Gritentnahme ausschließlich im vorderen Bereich des Rohrfermenters nicht zuverlässig.
Daher ist nach einem weiteren Merkmal der Erfindung ein über die Länge des Rohrfermenters mehrstufiger Gritaustrag durch eine Reihe von einzelnen, hintereinander angeordneten Gritausträgen vorgesehen. Dadurch können sämtliche sich über den Prozeß bildenden Ablagerungen abgeführt werden.
In großen Rohrfermentem ablaufende Prozesse bedürfen einer wohldosierten Entnahme von Grit an ausgewählten Stellen des Rohrfermenters.
Dazu ist nach einem anderen Merkmal der Erfindung vorgesehen, daß jeder Gritaustrag unabhängig ansteuerbar ist.
Um eine bessere Einmischung des dem Reaktor zugeführten biogenen Materials mit dem bereits im Rohrfermenter befindlichen Materials zu gewährleisten, ist nach einem weiteren Merkmal der Erfindung vorgesehen, daß der Eintrag für das organische Material in den Rohrfermenter radial erfolgt.
Insbesondere ermöglicht der erfindungsgemäße radiale Eintrag des organischem Materials bei spezifisch leichteren Flüssigkeiten diesem, sich sehr rasch einzumischen.
Es ist nach einem weiteren Merkmal der Erfindung im radialen Eintrittsbereich für das organische Material eine richtungsgebende Ablenkvorrichtung vorgesehen, wobei die richtungsgebende Ablenkvorrichtung ein Einlenkblech für das organische Material in den Rohrfermenter ist.
Nach einer weiteren Ausbildung der Erfindung ist das Einlenkblech an die Krümmung des Rohrfermenters angepaßt und erstreckt sich idealerweise über 1/3 seines Umfanges.
Bei den bekannten, derzeit am Markt üblichen Einlässen wird im allgemeinen mit zu hohen Geschwindigkeiten gefahren. Damit gelangt das Material zu tief in den Fermenter. Im Eintrittsbereich des Fermenters erfolgt somit keine Abarbeitung des eingebrachten biogenen Materials. Dieser Mangel wird durch die genannte erfindungsgemäße Ausbildung beseitigt.
Ein weiteres Merkmal der Erfindung ist es, daß der radiale Eintrag in den Rohrfermenter in 90° zur Längsachse desselben vorgesehen ist. Dadurch erfolgt eine Rotation des Mediums im vorderen Bereich des Rohrfermenters. Daraus resultiert eine für den Abarbeitingsprozeß besonders günstige strömungstechnische Belegung.

Erfindungsgemäß ist auch vorgesehen, daß die jeder Reaktorwelle zugeordneten Rührwerke im Rohrfermenter zusammen mit ihren Antrieben symmetrisch aufgebaut sind.
In die Reaktorwelle sind Paddel eingesetzt. Diese Paddel tragen im Bereich der Reaktorwand Rührschaufeln. Um ein ideales Abarbeiten des eingesetzten biogenen Materials zu gewährleisten, sind die Rührschaufeln in bzw. gegen die Transportrichtung des biogenen Materials neigbar ausgebildet.
Nach einem Merkmal der Erfindung ist dabei vorgesehen, daß die Rührschaufeln gegenüber der Reaktorwand eine Neigung von ±5° bis±10° aufweisen.
Die Neigung der Rührschaufeln steht dabei in Abhängigkeit von der Gesamtneigung des Rohrfermenters [ zur Austragsseite hin gegenüber der Horizontalen leicht nach oben ] sowie des eingesetzten Substrates. Als Faustregel gilt : Je zäher das zu verarbeitende Substrat, desto schräger stehen die Rührschaufeln.
Nach einer Weiterbildung der Erfindung umfaßt jedes Paddel zwei Tragstäbe, deren eines Ende mit der Rührwellen verbunden sind und deren anderes Ende die Rührschaufel trägt.
Zur Anpassung der Winkelstellung der Rührpaddel ist erfindunngsgemäß auch vorgesehen, daß die Rührschaufel am Paddel angelenkt ist.
Um die Kontinuität der Erwärmung des biogenen Materials durch das gemeinsame Rotieren von Rührwerk und Heizwerk zu gewährleisten, ist nach einem weiteren Merkmal der Erfindung vorgesehen, daß jedes Heizwerk mit der Reaktorwelle fest verbunden ist.
Ein anderes Merkmal der Erfindung ist es, daß über die Verbindung Heizwerk/ Reaktorwelle die Anspeisung und Steuerung des Heizwerkes erfolgt.
Um das Heizmedium in das Heizwerk zu verbringen, ist erfindungsgemäß vorgesehen, daß die Anspeisung des Heizsystems mit einem Heizmedium über die Reaktorwelle und den Wellenantrieb erfolgt und daß jedes Heizwerk ein mit der Reaktorwelle fest verbundenes Rohrsystem umfaßt.
Um eine Anpassung der Temperatur des Heizmediums an die gefahrenen Prozesse zu ermöglichen, ist nach einem weiteren Merkmal der Erfindung vorgesehen, daß als Heizmedium ein temperatursteuerbares Liquid oder Gas eingesetzt wird.
Die Wellenantriebe selbst sind nach einem abschließenden Merkmal der Erfindung jeweils an den Enden und außerhalb des Rohrfermenters vorgesehen.

Die Erfindung wird nun anhand eines Ausführungsbeispieles unter Zuhilfenahme der angeschlossenen Zeichnung näher beschrieben.
Es zeigen
- Fig. 1: einen Längsschnitt durch den erfindungsgemäßen Rohrfermenter;
- Fig. 2: einen Längsschnitt durch eine andere Ausbildung des erfindungegemäßen Rohrfermenters;
- Fig. 3: einen Schnitt gemäß Linie A-A des erfindungegemäßen Rohrfermenters nach Fig. 1,
- Fig. 4: einen Schnitt gemäß Linie B-B des erfindungsgemäßen Rohrfermenters nach Fig. 1 und
- Fig. 5: einen Schnitt gemäß Linie C-C des erfindungsgemäßen Rohrfermenters nach Fig. 1.

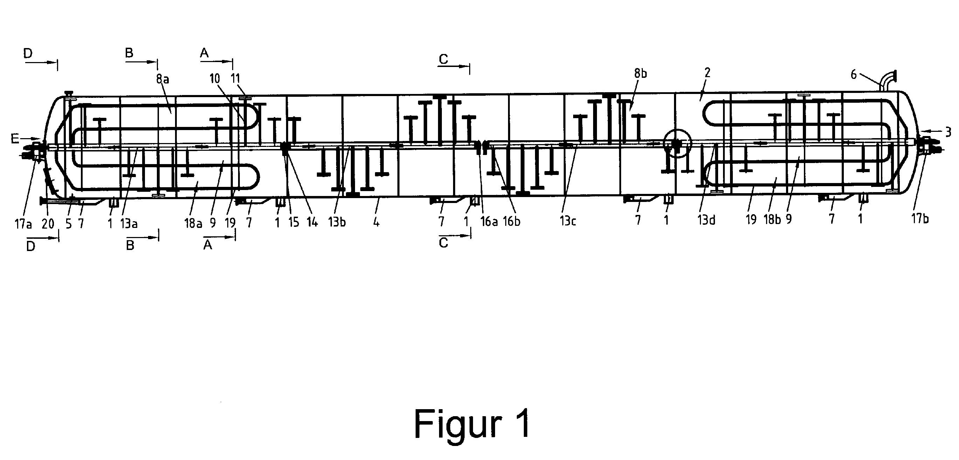

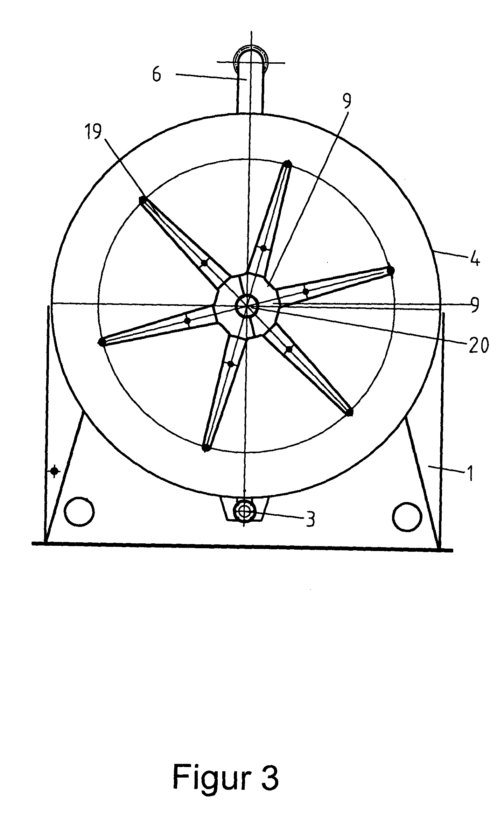

Fig. 1 zeigt einen auf mehreren Auflagerkonsolen 1 aufliegenden Rohrfermenter 2 in einem Längsschnitt. Der Rohrfermenter 2 ist zur Austragsseite 3 hin gegenüber der Horizontalen leicht nach oben geneigt angeordnet.
Das zu verarbeitende biogene Material wird dem Rohrfermenter 2 durch einen im Fermentermantel 4 angeordneten Eintrag 5 zugeführt. Der Materialeintrag erfolgt radial. Die radiale Zufuhr ermöglicht eine rasche Einmischung des neuen Materials mit dem bereits im Rohrfermenter 2 befindlichen Material. Eine spezielle Überimpfung ist hier nicht erforderlich. Der Abbau des biogenen Materials erfolgt in einer Pfropfenströmung.
Das bei der Abarbeitung des biogenen Materials entstehende Biogas wird über den Dom 6 abgeführt. In diesen ist ein nicht dargestellter Siphon zur Abnahme des vergorenen Endsubstrates integriert.
Abgesunkenes Material, insbesondere Sande, Steine, Glas, Metalle etc. sammeln sich am Fermenterboden und werden mehrmals täglich über den Gritaustrag 7 aus dem Rohrfermenter ausgebracht. Vorzugsweise werden die festen Anteile über mehrere Gritausträge 7 abgesaugt. Die Anzahl der Gritausträge 7 wird in Abhängigkeit von der Länge des Rohrfermenters 2 festgelegt. Jeder der Gritausträge ist unabhängig ansteuerbar.
Es sind zwei Rührwerke 8a,b vorgesehen. Jedes der Rührwerke 8a,b umfaßt eine Reaktorwelle 9 sowie eine Vielzahl von in diese eingesetzten Paddeln 10 mit aufgesetzten Rührschaufeln 11. Die Rührschaufeln 11 sind an den Paddeln 10 angelenkt. Dadurch können sie gegenüber dem Fermentermantel 4, also gegenüber der Innenseite des Fermenters 2 eine Stellung von ±5° bis±10° einnehmen. Die Stellung der Rührschaufeln 11 ist dabei abhängig von der Art des zu verarbeitenden Biosubstrates und der Schrägstellung des Fermenters 2 gegenüber der Horizontalen.
Jedes Paddel 10 ist durch zwei Tragstäbe 12 gebildet, deren eines Ende mit der Außenseite der Reaktorwelle 9 verbunden sind und deren anderes Ende die Rührschaufel 11 trägt.
Die Reaktorwelle 9 ist in Abhängigkeit von der Länge des Fermenters 2 in mehrere Wellenabschnitte 13a - 13d unterteilt. Zusammenhängende Wellenabschnitte 13a,b und 13c,d sind jeweils über Kupplungen 14 mitsammen verbunden. Die Kupplungen 14 selbst sind über je ein Stützlager 15 abgestützt. Die einander gegenüberliegenden Enden der Wellenabschnitte 13b und 13c sind durch jeweils ein Stützlager 16a,b unterstützt.
Die mitsammen verbundenen Wellenabschnitte 13a,b und 13c,d weisen einen eigenen Getriebemotor 17a,b auf. Jeder Getriebemotor 17a,b wird von einem nicht dargestellten Frequenzumrichter angesteuert. Das Durchmischen des Fermenterinhaltes kann somit intermittierend erfolgen, Vor- Rücklauf bzw. Schaukeln der Rührwerke ist möglich.
Mit der Reaktorwelle 9 bzw. den Reaktorwellenabschnitten 13a sowie 13d fest verbunden sind die Heizwerke 18a und 18b. Die Heizwerke 18a,b sind bezogen auf den Fermenter 2 symmetrisch angeordnet. Jedes der Heizwerke 18a,b umfaßt ein umfangreiches Rohrsystem, wie in Fig.3 dargestellt. Die das Heizmedium tragenden Rohre 19 erstrecken sich über ein Drittel der Länge der Reaktorwelle 9 in den Fermenter 2 hinein. Die Anspeisung des Heizwerkes 18a,b erfolgt über jeweils die zugeordnete Reaktorwelle 9 und den Getriebemotor 17a,b. Als Heizmedium ist ein temperaturgesteuertes Liqid oder Gas eingesetzt.

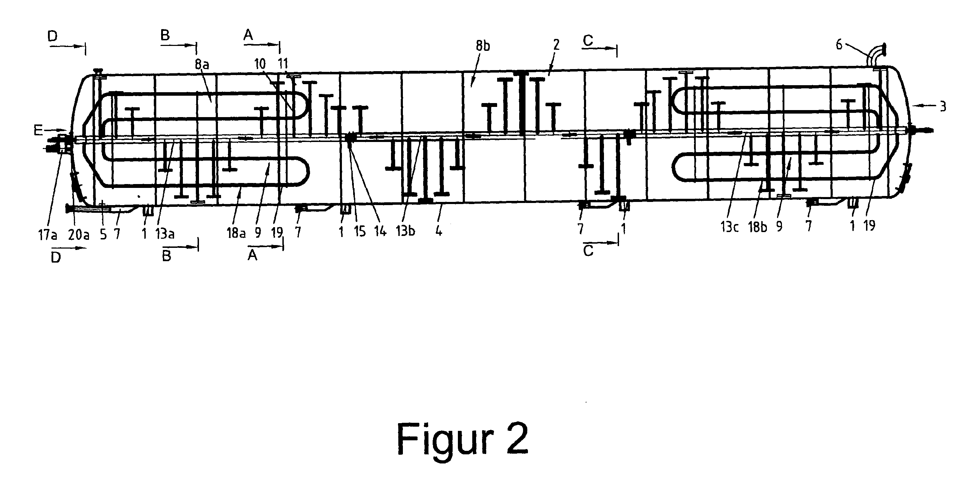

Fig.2 zeigt eine Ausbildung des Rohrfermenters mit einer aus drei Wellenabschnitten 13a, 13b, 13c bestehenden durchgehenden Reaktorwelle 9. Diese Ausführung verfügt lediglich über einen Getriebemotor 17a, jedoch ebenfalls über zwei Heizwerke 18a, 18b.

Fig.3 zeigt einen Schnitt entlang der Linie A-A der Fig.1.
Insbesondere ist hier die konstruktive Ausgestaltung eines der Heizwerke 18a dargestellt. Die Heizungsrohre 19 sind dabei sternförmig an der Reaktorwelle 9 angeordnet. Die Zufuhr des Heizmediums erfolgt über eine Zugangsleitung 20 in der Reaktorwelle 9. Die in der Reaktorwelle 9 vorgesehene Zugangsleitung 20 ist radial zweigeteilt ausgeführt. Im äußeren Bereich erfolgt der Heizungsvorlauf, im inneren Bereich erfolgt der Heizungsrücklauf, also der Rücklauf des Heizmediums.
Die Heizwerke 18a,b sind mit nicht dargestellten Sensoren versehen, welche eine stete Nachregulierung ermöglichen. Die Heizwerke 18a,b sind unabhängig voneinander ansteuerbar. Wärmeschwankungen, Ergänzung von Abstrahlwärme bzw. Realisierung von Prozeßtemperaturkurven sind somit leicht zu handhaben.

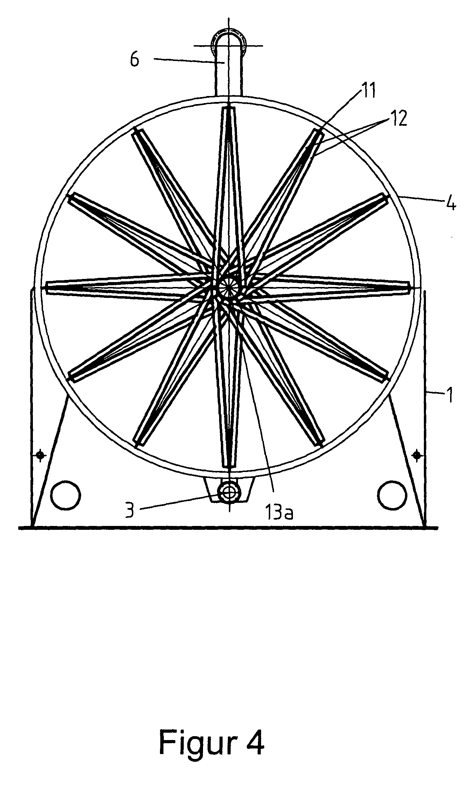

Fig.4 zeigt einen Schnitt entlang der Linie B-B der Fig.1 oder Fig. 2
Insbesondere ist hier die Anordnung und Ausbildung der Paddel 10 sowie der Rührwerkschaufeln 11 dargestellt.
Jedes Paddel 10 besteht aus zwei Tragstäben 12, welche jeweils mit der Reaktorwelle 9 fest verbunden sind. An der gemeinsamen Spitze der beiden Tragstäben 12 ist die Rührwerkschaufel 11 angelenkt.
Es sind 12 Paddel 10 vorgesehen. Deren Versetzung erfolgt somit in 12 Schritten oder einem Versetzungswinkel von 30°. Daraus bildet sich eine fiktive Schraube. Durch den Versetzungswinkel ist die in Fig.1 dargestellte Steigung der Schraube festgelegt. Das kontinuierliche Weiterschieben des Materialpfropfens durch die angelenkten Rührwerkschaufeln 11 wird durch deren Schrägstellung in Abhängigkeit vom Material unterstützt. Ergänzend dazu erfolgt die Neigung des Rohrfermenters 2 ebenfalls in Abhängigkeit vom zu verarbeitenden Substrat.

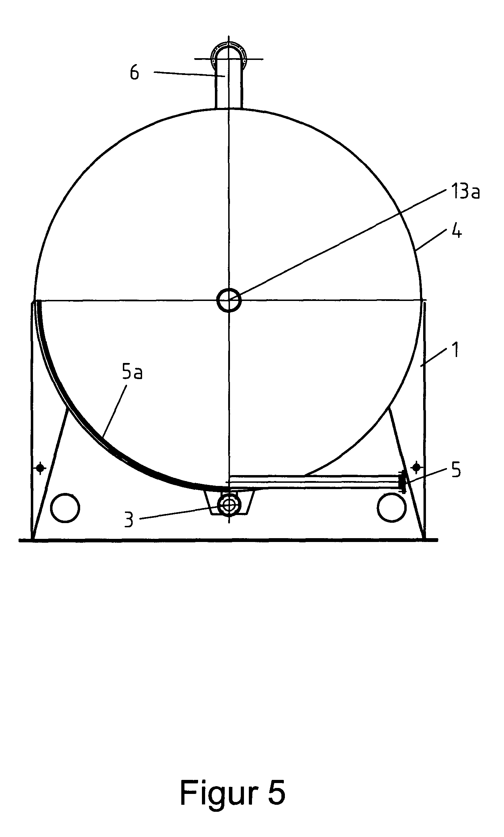

Fig.5 zeigt einen Schnitt entlang der Linie D-D der Fig.1 oder Fig. 2.
Hier ist insbesondere der Weg des biogenen Materials in den Rohrfermenter 2 nachzuvollziehen.
Das biogene Material gelangt über den Eintrag 5 in den Rohrfermenter 2. Dort wird es über das Einlenkblech 5a durch Rotation eingeführt. Das Einlenkblech 5a erstreckt sich über ein Drittel des Rohrfermenterumfanges und entspricht im wesentlichen dessen Radius.

## Patentansprüche

1. Rohrfermenter zur Fermentation organischen Materials unter Erzeugung von Biogas, mit einem Eintrag (5) für das organische Material und einem Austrag (6) für das organische Material und Biogas, einem Rührwerk (8a, 8b), einer mit diesem umlaufenden Heizung sowie einem Gritaustrag (7), **dadurch gekennzeichnet, daß** die Heizung zwei oder mehrere voneinander unabhängig steuerbare Heizwerke (18a, 18b)umfaßt und daß jedes der Heizwerke 18a, 18b) einem vorwählbaren Bereich des korrespondierenden Rührwerkes (8a, 8b) zugeordnet ist.

2. Rohrfermenter nach Anspruch 1, mit einem aus Reaktorwelle (9), einer Vielzahl über die Längserstreckung der Reaktorwelle (9) gegeneinander versetzter Paddel (10) sowie Rührschaufeln (11) im Bereich der Reaktorwand bestehenden Rührwerk (8a,8b), **dadurch gekennzeichnet, daß** die Reaktorwelle (9) aus wenigstens zwei- oder mehreren Wellenabschnitten (13a, 13b, 13c, 13d) besteht.

3. Rohrfermenter nach Anspruch 2, **dadurch gekennzeichnet, daß** zwei unabhängige Reaktorwellen (9) vorgesehen sind und daß jede Reaktorwelle (9) einen unabhängigen Wellenantrieb (17a, 17b) aufweist.

4. Rohrfermenter nach einem der Ansprüche 1 oder 2, **dadurch gekennzeichnet, daß** eines der Heizwerke (18a) im Eintrittsbereich des Rohrfermenters (2) vorgesehen ist.

5. Rohrfermenter nach Anspruch 4, **dadurch gekennzeichnet, daß** das zweite sowie jedes weitere Heizwerk (18b) im an den Eintrittsbereich anschließenden Bereich des Rohrfermenters (2) vorwählbar vorgesehen ist [ sind ].

6. Rohrfermenter nach Anspruch 1, **dadurch gekennzeichnet, daß** jedes der Heizwerke (18a, 18b) partiell ansteuerbar ist.

7. Rohrfermenter nach Anspruch 1, **dadurch gekennzeichnet, daß** der Gritaustrag (7) außerhalb des Bereiches des Eintrages (5) für biogenes Material angeordnet ist.

8. Rohrfermenter nach Anspruch 7, **dadurch gekennzeichnet, daß** der Gritaustrag (7) eine aus dem Rohrfermenter (2) schräg nach unten verlaufende Austragstrecke umfaßt.

9. Rohrfermenter nach Anspruch 2, **dadurch gekennzeichnet, daß** die Rührschaufeln (11) gegenüber der Reaktorwand eine Neigung von ±5° bis±10° aufweisen.

10. Rohrfermenter nach Anspruch 1, **dadurch gekennzeichnet, daß** der Eintrag (5) für das organische Material in den Rohrfermenter (2) radial erfolgt.

11. Rohrfermenter nach Anspruch nach den Ansprüchen 1, 7 oder 8, **dadurch gekennzeichnet, daß** über die Länge des Rohrfermenters (2) ein mehrstufiger Gritaustrag (7) durch eine Reihe von Gritausträgen vorgesehen ist.

12. Rohrfermenter nach Anspruch 11, **dadurch gekennzeichnet, daß** jeder Gritaustrag (7) unabhängig ansteuerbar ist.

13. Rohrfermenter nach Anspruch 2, **dadurch gekennzeichnet, daß** die Enden jeweils zweier benachbarter Wellenabschnitte (13a,13b;13c,13d) von einem gemeinsamen Stützlager (15) geführt sind.

14. Rohrfermenter nach Anspruch 3, **dadurch gekennzeichnet, daß** die Enden der aus Wellenabschnitten (13a,13b;13c,13d) gebildeten Reaktorwellen (9) in Stützlagern (16a,16b) geführt sind.

15. Rohrfermenter nach Anspruch 1, **dadurch gekennzeichnet, daß** jedes Heizwerk (18a,18b) mit der Reaktorwelle (9) fest verbunden ist.

16. Rohrfermenter nach Anspruch 15, **dadurch gekennzeichnet, daß** über die Verbindung Heizwerk (18a,18b) - Reaktorwelle (9) die Anspeisung und Steuerung des Heizwerkes (18a,18b) erfolgt.

17. Rohrfermenter nach Anspruch 16, **dadurch gekennzeichnet, daß** die Anspeisung des Heizwerkes (18a,18b) mit einem Heizmedium über eine Zugangsleitung (20) in der Reaktorwelle (9) und den Wellenantrieb (17a, 17b) erfolgt.

18. Rohrfermenter nach einem der Ansprüche 15-18, **dadurch gekennzeichnet, daß** jedes Heizwerk (18a, 18b) ein mit der Reaktorwelle (9) fest verbundenes Rohrsystem (19) umfaßt.

19. Rohrfermenter nach Anspruch 17, **dadurch gekennzeichnet, daß** als Heizmedium ein temperatursteuerbares Liquid oder Gas eingesetzt wird.

20. Rohrfermenter nach Anspruch 3, **dadurch gekennzeichnet, daß** die Wellenantriebe (17a, 17b) jeweils an den Enden und außerhalb des Rohrfermenters (2) vorgesehen sind.

21. Rohrfermenter nach Anspruch 3, **dadurch gekennzeichnet, daß** die jeder Reaktorwelle (9) zugeordneten Rührwerke (8a,8b) im Rohrfermenter (2) zusammen mit ihren Antrieben symmetrisch aufgebaut sind.

22. Rohrfermenter nach Anspruch 2, **dadurch gekennzeichnet, daß** jedes Paddel (10) zwei Tragstäbe (12) umfaßt, deren eines Ende mit der Reaktorwelle (9) verbunden sind und deren anderes Ende die Rührschaufel (11) trägt.

23. Rohrfermenter nach Anspruch 2 oder 22, **dadurch gekennzeichnet, daß** die Rührschaufel (11) am Paddel (10) angelenkt ist.

24. Rohrfermenter nach Anspruch 10, **dadurch gekennzeichnet, daß** im radialen Eintrittsbereich für das organische Material eine richtungsgebende Ablenkvorrichtung (5a) vorgesehen ist.

25. Rohrfermenter nach Anspruch 24, **dadurch gekennzeichnet, daß** die richtungsgebende Ablenkvorrichtung (5a) ein Einlenkblech für das organische Material in den Rohrfermenter (2) ist.

26. Rohrfermenter nach Anspruch 25, **dadurch gekennzeichnet, daß** das Einlenkblech an die Krümmung des Rohrfermenters (2) angepaßt ist.

27. Rohrfermenter nach den Ansprüchen 25 oder 26, **dadurch gekennzeichnet, daß** sich das Einlenkblech über 1/3 des Umfanges des Rohrfermenters (2) erstreckt.

28. Rohrfermenter nach einem der Ansprüche 10 oder 24, **dadurch gekennzeichnet, daß** der radiale Eintrag (5) in den Rohrfermenter (2) in 90° zur Längsachse desselben vorgesehen ist.
